Europäisches Patentamt

European Patent Office

Office européen des brevets

(11) Publication number: **0 274 911 B1**

# EUROPEAN PATENT SPECIFICATION

(45) Date of publication of patent specification: **03.03.93**

(51) Int. Cl.⁵: **G01N  33/52**, G01N 31/22,
//G01N33/564,G01N33/545,
G01N33/577,G01N33/534

(21) Application number: **87311509.1**

(22) Date of filing: **30.12.87**

(54) **Diagnostic device, method for making, and method for using.**

(30) Priority: **12.01.87 US 2215**

(43) Date of publication of application:
**20.07.88 Bulletin  88/29**

(45) Publication of the grant of the patent:
**03.03.93 Bulletin  93/09**

(84) Designated Contracting States:
**DE FR IT SE**

(56) References cited:
EP-A- 0 005 536     EP-A- 0 087 228
EP-A- 0 090 483     EP-A- 0 154 839
EP-A- 0 173 500     DE-A- 3 118 381
US-A- 4 693 985

(73) Proprietor: **PALL CORPORATION**
**30 Sea Cliff Avenue**
**Glen Cove New York 11542(US)**

(72) Inventor: **Degen, Peter John**
**24 Glades Way**
**Huntington, NY 11743(US)**
Inventor: **Gsell, Thomas C.**
**40 Valentine Avenue**
**Glen Cove, NY 11542(US)**
Inventor: **Matkovich, Vlado I.**
**11 Old Estate Road**
**Glen Cove, NY 11542(US)**
Inventor: **Martin, Jerold**
**250 Mercer Street, Apt. C619**
**New York, NY 10012(US)**

(74) Representative: **Corin, Christopher John et al**
**Mathisen Macara & Co. The Coach House 6-8**
**Swakeleys Road**
**Ickenham Uxbridge UB10 8BZ (GB)**

EP 0 274 911 B1

**Description**

This invention relates to a device for performing clinical diagnostic tests and methods for carrying out these tests. Specifically, it relates to a device and methods for detecting and quantifying the presence of biologically-active materials. More specifically, this relates to a device comprising a microporous membrane, on which detection reagents are immobilized, which membrane is bonded to an inert support, enabling convenient handling of the device as it is inserted into and removed from materials to be analyzed. The invention also relates to a method for making diagnostic devices.

Modern medical technology is heavily dependent on the ability to identify the presence of harmful organisms, antibodies, enzymes, toxins, genetic material, and the like in biological specimens. Analytical procedures are presently in use which can detect simple chemical substances, such as drugs, sugar, and metabolites (biochemical breakdown products) of biological materials. These procedures are usually performed by standard chemical analytical procedures, many of which require large quantities of specimen and difficult, time-consuming conversion of the specimen into a form which can be analyzed by chemical or instrumental methods, such as by colorimetric analysis, by chromatography, or by mass spectrometry.

Recently, test kits have been introduced whereby the presence of simple chemicals, such as sugar, or even more complex materials such as enzymes, can be estimated in biological fluids, e.g., blood or urine. Such kits consist of absorbent materials which are impregnated with a reagent or a combination of reagents which produce a change in color in the presence of certain biological substances. In these tests, the matrix is first immersed in the fluid in order to contact the reagents with the substance in the fluid of interest. Then, the matrix is removed from the liquid and a color change is noted. In many cases, the matrix must be washed or developed with a series of reagents to cause a visible reaction. The visible change indicates the presence of the biological chemical sought. Such test kits are used, e.g., to detect glucose in urine and to detect phenylketonuria (phenylpyruvic oligophrenia or PKU).

Such test kits are useful because they often require only small amounts of the fluid to be tested (specimen), little preparation of the specimen, and the analysis is usually rapid. However, the results of such tests are not quantitative, except in certain cases where the substance to be analyzed is present in relatively large amounts, for example, glucose in urine.

More recently, advances in immunology, genetics, and biotechnology have yielded scientific techniques for detecting exceedingly small amounts of biologically-active substances, such as enzymes, hormones, antibodies, antigens, and genetic matter. Detection of such substances and the ability to quantify them would enable the rapid diagnosis of many diseases. Further, quantification would enable both differential diagnosis among diseases and determination of whether or not the disease is active.

For example, small amounts of a toxin can be detected by the following method. The material to be analyzed can be exposed to a matrix on which an antibody to the toxin is immobilized. An antibody is a protein produced by living cells of an animal which has a specific affinity for another (usually foreign) biological substance with which the animal may come in contact. Any of this toxin in the specimen will then form an affinity complex with the antibody on the matrix. Because antibodies are specific for the substance with which they complex, only the toxin whose antibody is immobilized on the matrix will bind to it. No other toxins or other materials in the specimen will form a complex with the antibody on the matrix. Therefore, the specimen does not need to be purified or converted to a more convenient form prior to analysis.

Once the toxin is bound to the antibody-laden matrix, the matrix can then be exposed to a solution containing another antibody to the toxin. This antibody must have first been modified with a chemical marker or "label" which enables it to be easily detected. The label could be a radioactive atom, a fluorescent dye, or an enzyme which can react with other chemicals to produce a visible color. The labeled antibody will bind to the matrix only in the amount that the toxin was bound to the matrix in the previous step because the labeled antibody binds only to the toxin.

In such an analysis, the labeled antibody can be easily detected and quantified. Because it is present in the same amount that the toxin is present, the toxin is also quantified by this method. Several variations of this method are known, all of which are based on the abilities of antibodies to form specific complexes with certain other biological substances (referred to as "antigens"). Such tests are able to detect and diagnose such diseases as serum hepatitis. The technology now exists to produce large quantities of antibodies which are specific to antigens of many types. Therefore, it should be possible to create diagnostic test equipment to detect a great many clinically important substances.

This type of analysis represented a major advance over the prior art in that the effort required to prepare the sample was reduced and the lower limit of detection was often improved. However, there are major drawbacks to devices currently utilizing this technique. These drawbacks are: (1) existing devices have limited sensitivity because the support matrices used are not optimal for the materials to be used in the test, and (2) existing diagnostic test devices are not quantitative. The present invention overcomes both of these drawbacks and represents a significant advance over the existing art.

This invention is directed to a diagnostic device for testing liquid to quantitatively detect the presence of biologically-active material in the liquid. The diagnostic device comprises an elongated support member having secured to at least a portion thereof a liquophilic, microporous, synthetic, polymeric membrane indicator medium or test strip, the membrane having a surface area of from 2 to 12 $m^2$/gram, a pore rating of from 0.04 to 10 micrometers with a pore size distribution of no greater than ± 15% and the ability to immobilize the reagent thereon in a reproducible manner. The polymeric membrane is an alcohol-insoluble polyamide membrane or a polyvinylidene difluoride membrane.

The invention is also directed to a diagnostic method for testing a liquid to quantitatively detect biologically-active material in the liquid comprising contacting a liquophilic, microporous, synthetic, polymeric membrane indicator medium or test field secured to at least a portion of an elongated support member and having a reagent immobilized thereon with the liquid containing the biologically-active material to be analyzed for, the membrane having a surface area of from 2 to 12 $m^2$/gram, a pore rating of from 0.04 to 10 micrometers with a pore size distribution of no greater than ± 15% and the ability to immobilize the reagent thereon in a reproducible manner.

The invention is further directed to a method of preparing a diagnostic device for biologically-active materials comprising treating an indicator medium or test field material to immobilize one or more test reagents thereon and thereafter preparing a diagnostic device from a portion of the treated indicator medium or test field material.

The tests based on antibody-antigen interaction and tests based on identification of genetic material (DNA) each involve the reaction of two reagents. In the first case, the reaction takes place between antibodies and antigens and, in the second case, between DNA and gene-specific chemicals referred to as "DNA probes". In each case, one component of the reactive system - an antibody, an antigen, or a DNA probe - is a small molecule that can penetrate the pore structure of most matrices which can be used as supports. However, the second component - an antigen or genetic matter - can be present in forms which vary in size from molecular dimensions to whole cells several microns in diameter.

As will be discussed in detail below, the materials selected as the porous support matrix, i.e., the indicator medium or test field, must meet several criteria. These criteria are: (1) a predetermined, high total available surface area, (2) a carefully defined and controlled pore size and pore size distribution, (3) liquophilicity, preferably hydrophilicity, and (4) the ability to immobilize, in a reproducible manner, the selected reagent.

To maximize the sensitivity of the diagnostic device, it is essential that all materials completely penetrate the porous support matrix, i.e., the indicator medium or test field. Therefore, the pore size of the support must be large enough for the largest reagents - antigens or genetic matter - to penetrate the support. It is also important to maximize the surface area of the indicator medium to enable immobilization of the largest amount of reagent as possible to enhance sensitivity. As employed herein, the term "surface" or "surface area" refers not only to the gross surface(s) of the medium, but also to the surfaces of the micropores of the medium, i.e., the interior surfaces which are contacted by fluid during use. Available surface area decreases as pore size increases. Accordingly, for any given system of reagents, the optimum pore size for the support should be the smallest pore size which will permit complete penetration of the largest reactant. This size will vary from submicron dimensions when the largest reactant is a virus, an enzyme, or an antibody to several microns when the largest reactant is a whole cell or a cell fragment. The ease and completeness of penetration is also effected by the wetting characteristics of the indicator medium as discussed below.

In order for a diagnostic procedure to yield quantitative results, the amounts of reagents delivered to the system must be fixed and reproducible in each test. The indicator medium or test field of a diagnostic device should, therefore, be capable of having immobilized on its surface, in a uniform manner, a fixed amount of reagent per unit volume of indicator medium or test field (membrane). By a "unit volume" of the medium is meant the volume occupied by a section of the medium (membrane), i.e., a given planar membrane area and thickness. Within that unit volume of the indicator medium is a voids volume, e.g., 70%, which is available for penetration of the reagent - containing liquid medium. For a fixed pore size, the higher the voids volume, the greater will be the surface area per unit volume of the indicator medium available for immobilization of the medium, hence the greater the test sensitivity. The surface area of the indicator medium or test field of the unit volume, as noted above, must be high to immobilize the largest amount of reagent as possible to enhance sensitivity. Further, to provide quantitative results, the surface area of the membrane in that unit volume, as well as the balance of the indicator medium or test field, must be uniformly distributed to insure uniform immobilization of the reagent.

3

The total amount of the reagent immobilized will be determined by the binding capacity per unit volume of the indicator medium or test field and by its total volume. The reproducibility of the test will depend on the uniformity of the medium. Therefore, to provide a diagnostic device with quantitative results, high sensitivity, and good reproducibility, it is necessary to use a fixed amount of reactant immobilized in a uniform manner on a fixed volume of uniform medium with high binding capacity, whose pore size has been determined to meet the requirements set forth above, i.e., the pore size must be large enough to allow access of the largest reagent while providing adequate surface area, bearing in mind that when pore size increases, surface area decreases. Materials found to be useful as the indicator medium or test field in the diagnostic devices in accordance with this invention are liquophilic, microporous membranes as described in more detail below.

The indicator medium or test field (membrane) must not react adversely with substances found in either the samples, reagents, or solvents employed in the analyses. Further, the membranes used should be essentially free of extractables which would adversely affect the particular test being carried out. To ensure good penetration and uniform distribution of the test reagents, the membrane must be liquophilic, i.e., spontaneously wettable by the test fluids. In addition, for the reasons discussed above, the indicator medium or test field must have a high surface area coupled with a pore size which will allow penetration of the largest test reagent or reactant. Membranes with surface areas of at least 2 $m^2$/gram and up to 12 $m^2$/gram or even higher, coupled with a pore size or pore rating in the range of from 0.04 to 10 microns, and most preferably 0.1 to 5 microns, are satisfactory. Pore ratings of the membranes useful as the indicator medium or test field in accordance with the subject invention are determined by measuring the $K_L$ of the membrane. The $K_L$ is essentially equivalent to bubble point, foam-all-over point, and other similar techniques familiar to those skilled in the art.

Pore rating is related to $K_L$ by the formula:

$$D = \frac{C}{K_L}$$

where D is the pore rating in micrometers, $K_L$ is the measured $K_L$ in psi, and C is a constant, which has a value of about 10 when using water as the wetting fluid for $K_L$ measurement. This relation is described in Publication NM 900a, dated September 1980, available from Pall Corporation. The pore size distribution of the membranes used as the indicator medium or test field typically have pore size distributions of no more than ± 15% of the pore rating determined in the manner described above, preferably of no more than ± 10%.

Membranes which are suitable for use as the indicator medium or test field typically have voids volumes in the range of 60 to 90%, preferably in the range of 70 to 90%. Preferred materials are hydrophilic in nature and are, therefore, easily water-wettable and tend to freely pass aqueous solutions.

Liquophilic, microporous membranes useful in accordance with the present invention include alcohol-insoluble polyamide membranes and polyvinylidene difluoride membranes which have been rendered liquophilic by appropriate treatment, such as by surface modification. As used herein, included within the term alcohol-insoluble polyamide membranes are modified polyamide membranes, containing as a major component thereof, an alcohol-insoluble polyamide.

Liquophilicity, as used herein, refers to the spontaneous wettability of the membrane by the liquid(s) with which it is contacted. The wettability or liquophilicity of a structure, e.g., a membrane, is a function of that structure's critical surface energy and the surface tension of the applied liquid. If the critical surface energy is at least as high as the surface tension of the liquid, then the liquid will spontaneously wet the structure. For example, a microporous membrane having a critical surface energy of 0.72 N/M (72 dynes/cm) or higher will be wetted by water which has a surface tension of 0.72 N/M (72 dynes/cm), i.e., the membrane is hydrophilic.

Suitable membranes must be capable of being treated with and retaining or immobilizing the reagent used to perform a specified test or reaction with the substance being analyzed for in a sample. As noted above, to obtain quantitative results, the reagent must be immobilized on the surface of the membrane in a uniform manner, in a fixed amount, and in a reproducible manner. The necessity for uniform immobilization and high sensitivity dictates the use of a uniform, homogeneous membrane and precludes the use of a fibrous/resin-based system as the indicator medium due to (1) the non-homogeneity of the system, (2) the inherent low surface area of the fibers, and (3) the non-discriminating pore sizes of such fiber-based systems with widely varying pore sizes resulting from random crossing of fibers. The reactant, which may be of ionic, molecular, or macromolecular nature, may be immobilized on the reaction layer by strong physical forces or by being bonded in some manner, such as by covalent chemical coupling, to the surface of the membrane layer.

As noted, wettability or liquophilicity is a requisite of the membranes used as the indicator medium or test field in accordance with the present invention to (1) ensure complete and uniform penetration of the reagent-containing liquid and, hence, full use of the membrane and uniform immobilization of the reagent on the surface of the membrane, as well as (2) complete and uniform penetration of the liquid containing the analyte, i.e., the substance being analyzed for. Preferred membranes used as the indicator medium or test field in accordance with the present invention are intrinsically hydrophilic or water-wettable.

The unique high-binding capacity, uniformity, controlled pore size, and high surface area of the hydrophilic, microporous nylon 66 membranes described in U.S. Patent 4,340,479 and available commercially from Pall Corporation under the trademark BIODYNE® A make them particularly useful as the indicator medium or test field. Basically, the hydrophilic, microporous, polyamide filter membranes disclosed in U.S. Patent 4,340,479 are membranes prepared from alcohol-insoluble polyamide resins having a methylene to amide ratio in the range of 5:1 to 7:1. Membranes of this group include copolymers of hexamethylene diamine and adipic acid (nylon 66), copolymers of hexamethylene diamine and sebacic acid (nylon 610), homopolymers of poly-ε-caprolactam (nylon 6), and copolymers of hexamethylene diamine and azelaic acid (nylon 69).

Another preferred membrane is POSIDYNE®, also available commercially from Pall Corporation. POSIDYNE® is a hydrophilic, microporous, skinless nylon 66 membrane with the surface properties controlled by quaternary ammonium groups of a polyamine-epichlorohydrin polymer or resin cocast with the nylon 66.

Another preferred membrane is IMMUNODYNE™, available from Pall Corporation. IMMUNODYNE™ is a modified CARBOXYDYNE® membrane, also available from Pall Corporation. CARBOXYDYNE® is a hydrophilic, microporous, skinless nylon 66 membrane with controlled surface properties formed by the cocasting process described in U.S. Patent 4,707,266, as discussed below, specifically by cocasting nylon 66 and a polymer containing an abundance of carboxyl groups to form a membrane having controlled surface properties characterized by carboxyl functional groups at its surfaces. IMMUODYNE™ membranes may be prepared from CARBOXYDYNE® membranes by treating them with trichloro-s-triazine in the manner described in U.S. Patent 4,693,985, discussed below.

Polyvinylidene difluoride membranes are not inherently water-wettable, but can be rendered such by an appropriate surface treatment. Microporous, polyvinylidene difluoride membranes which have been treated to render them hydrophilic are commercially available, e.g., from Pall Corporation under the trademark FLUORODYNE™ and from Millipore Corporation as hydrophilic DURAPORE® As discussed above, wettability or liquophilicity is a function of the critical surface energy of the structure and the surface tension of the liquid. Wettability may also be expressed in terms of intrusion pressure required for liquid to penetrate into the pores of the membrane. Although a function of the properties of the liquid used, such as surface tension, membrane materials which are particularly preferred have intrusion pressures of, or close to, zero.

As noted above, membranes useful as the indicator medium have large surface areas, typically from 2 to 12 $m^2$/gram or even higher. This characteristic permits a greater amount or higher concentration of reactant to be immobilized in the indicator medium. Accordingly, higher sensitivities may be achieved using the diagnostic device in accordance with the present invention.

The polyamides preferred for use in accordance with the present invention include alcohol-insoluble nylons of the type described in U.S. Patent 4,340,479. As noted above, a membrane material of this description which is particularly useful for the present invention is a microporous, hydrophilic nylon membrane (nylon 66) commercially available from Pall Corporation under the trademark BIODYNE®.

Also included among the preferred polyamide membranes for the present invention are hydrophilic, microporous, skinless, alcohol-insoluble polyamide membranes with controlled surface properties of the types described in U.S. Patent 4,707,266 and in U.S. Patent 4,702,840. These hydrophilic, microporous, substantially alcohol-insoluble polyamide membranes with controlled surface properties are formed by cocasting an alcohol-insoluble polyamide resin with a water-soluble, membrane-surface-modifying polymer having functional polar groups. Like the preferred hydrophilic, microporous, nylon membranes which do not have controlled surface-modified polar groups present, the polyamide membranes used as the indicator medium or test field in the present invention having controlled surface properties are also skinless; that is, they are characterized by through pores extending from surface-to-surface which are of substantially uniform size and shape. If desired, however, materials having tapered through pores, i.e., pores which are larger at one surface of the sheet, narrowing as they approach the opposite surface of the sheet, may be used.

The surface-modifying polymers used to prepare the polyamide membranes with controlled surface properties, useful in accordance with the present invention, comprise polymers which contain substantial proportions of chemically functional groups, such as hydroxyl, carboxyl, amine, and imine groups. As a result, the membranes include, at their surfaces, high concentrations of functional groups such as hydroxyl, carboxyl, amine, imine, or a combination of any of the above groups which do not react with one another. These polyamide membranes having controlled surface properties have higher concentrations of carboxyl or imine groups at their surfaces than the other preferred alcohol-insoluble, microporous, hydrophilic, skinless polyamide membranes described above which do not have controlled surface properties, i.e., those which are formed from the preferred polyamide resin but are not cocast with surface-modifying polymer.

The membranes used may be treated by any method known to one of skill in the art to deposit and/or bind reagents thereto. As indicated above, the reagent may be of an ionic, molecular, or macromolecular nature. When used as a diagnostic tool to provide a visible change, the reagent may be one or a combination of substances which is initially colorless and which, upon reaction with a suitable material, provides an optically measurable response. Other possible variations include the use of suitable labels, such as the formation between the deposited reagent and the material for which testing is being conducted of a complex or compound which is appropriately labeled by any known technique, such as enzymatic/substrate labels or the like.

Treatment of the indicator medium or test field (membrane) of the diagnostic device with a suitable reagent(s) may be performed at the time at which diagnostic tests are to be performed. Specifically, the test reagent(s) may be added immediately preceding contacting of the test device with the liquid containing the sample to be analyzed for, e.g., by "spotting" the reagent(s) on the membrane secured to the support member. However, the present invention is expected to have greatest application to, and a preferred embodiment includes, diagnostic devices prepared by treating indicator medium or test field material with one or more test reagents to immobilize one or more test reagents thereon, and thereafter preparing the individual diagnostic devices from a portion of the treated material. For example, a ribbon or continuous strip of a membrane may be treated with the reagent(s) and then that membrane applied to the material to be used as the support member following which individual diagnostic devices are manufactured. This will be more clear be reference to Example 1 below. Other variants include (1) placing a treated ribbon of membrane on top of a second wider untreated ribbon of the same membrane to provide a controlled background medium prior to manufacturing of the individual diagnostic devices and (2) applying a band of the reagent(s) to a ribbon of membrane to provide the controlled background within a single piece of membrane.

A useful method of binding reagents of a molecular nature, especially macromolecules, and particularly those of a biological nature, is disclosed in U.S. Patent. 4,693,985. This patent describes a method for immobilizing a wide range of biologically active substances as acceptor molecules on active membranes. The acceptor-bound membranes described in the patent are capable of immobilizing and binding a wide variety of biologically-active compounds, specifically ligands, to the acceptor molecules. Using such reaction layers or membranes permits the testing of bodily fluids, such as blood, serum, plasma, urine, salivas, and the like. It also permits testing for particular substances by chemical assays or immunoassays, such as those where a specific label is employed, such as one indicating enzyme activity, radioactivity, or an electromagnetic energy absorbing and/or emitting label, such as a fluorescent label. The macromolecules used as reagents and bound to the surfaces of the microporous membrane or which are assayed for using the device in accordance with the present invention generally include materials of a biological nature and are frequently proteinaceous in nature. The reagent or acceptor molecule bound directly to the reaction layer or the ligand being tested for include immunoglobulins or antibodies (either polyclonal or monoclonal), antigenic substances, apoproteins, receptors, glycoproteins, lectins, carbohydrates, hormones, enzymes, carrier proteins, heparin, coagulation factors, enzyme substrates, inhibitors, cofactors, nucleic acids, etc.

It should be understood that a variety of diagnostic devices for testing liquids to detect the presence of a substance in that liquid may be used. For example, the diagnostic device or test strip may comprise an elongated strip or support member having sufficient rigidity to allow the test strip to be handled and a test field or indicator medium secured at or near one end of the support member, i.e., near the end opposite that end used as a handle. Alternatively, a second test field or indicator medium may be attached to the side of the support member opposite that to which the test field or indicator medium is attached.

Also, a test field or indicator medium which is, e.g., approximately one-half the width of the support member, may be secured to a background, untreated medium useful for comparison purposes, i.e., as a baseline or reference.

6

Also, an aperture may be formed near one end of the elongated strip or support member in which the test field or indicator medium is secured. Such a configuration provides ease of liquid penetration and rinsing.

A still further embodiment is a test device in which an aperture is formed in the support member in which is secured a composite test field or indicator medium characterized by two or more layers. In such a configuration, one layer may be an untreated medium to act as a reference or baseline with the other layer, the treated test field or indicator medium. In operation, such a configuration may be read optically by reading first one side, e.g., the reference or baseline side, and then the other side.

The elongated support member used in accordance with the subject invention may be made from a variety of materials and take a variety of forms. The material will typically have an elongated, rectangular shape of, for example, 6.4 cm (2.5 inches) to 10.2 cm (4 inches) in length, and 0.5 cm (0.2 inch) to 1.0 cm (0.4 inch) in width. Preferably, it is a polyester or cellulose acetate film of, for example, 0.2 mm (8/1,000 inch) to 0.4 mm (15/1,000 inch) thick.

Alternatively, polypropylene or the like may also be used. Basically, the criteria for selection of the support member is that it provide sufficient rigidity to allow handling of the test device and that it not interact in any manner with the test liquid, i.e., that it be inert.

The invention will be better understood by reference to the following examples which are offered by way of illustration and not by way of limitation.

## Example 1

A device for rapidly and quantitatively detecting the antibody to the bovine hormone glucagon was fabricated in the following manner.

An IMMUNODYNE™ membrane (a commercially available product of Pall Corporation as described above) having a pore size of 0.2 micrometer and a BET surface area of 8.44 $m^2$/gram was first loaded with glucagon in a controlled fashion as described below. One hundred milligrams glucagon (Product No. 345995, a product of Calbiochem Inc.), was dissolved in 1 milliliter of a 0.1 N aqueous sodium hydroxide solution. Ten microliters ($\mu$l) of this solution was then added to 100 ml of a solution of a 0.01 M sodium phosphate buffer (pH 7.0) having a concentration of 0.15 M sodium chloride (hereinafter "phosphate buffered saline or "PBS") to make a solution containing 10 $\mu$g/ml glucagon. A 7.6 cm (3 inch) by 20.3 cm (8 inch) piece of IMMUNODYNE™ was immersed in 10 ml of this solution. The membrane was wetted immediately upon contact with the solution, indicating that the liquid filled the void volume of the membrane in its entirety and all internal pore surfaces were contacted. The membrane was allowed to remain in contact with the solution for one hour to effect chemical binding, after which the membrane was dried in an oven at 40°C for 1/2 hour. The membrane was then immersed in 15 ml of a solution containing 0.5 grams of casein per 100 ml of PBS for 1 hour, washed twice by agitation in successive 25 ml portions of PBS and dried again in an oven at 40°C for 1/2 hour.

A 180 mm side roll of 254 micrometers (10 mil) thick cellulose acetate film was coated on one side with a 32 mm wide band of MA-27, an acrylic pressure sensitive adhesive available from Adhesive Research, Inc. The band was applied at a coating weight of 3.2 $g/m^2$ (0.95 oz/yd$^2$) at the center of the film while running lengthwise above the roll as it was unwound. To protect the structure during handling, the layer of adhesive was covered with a protective layer of release paper which was siliconized on both sides to effect easy removal or release from the adhesive.

After subsequent removal of the release paper, a 32 mm wide strip of the IMMUNODYNE™ membrane loaded with glucagon was applied to a 20.3 cm (8 inch) length of the coated cellulose acetate film directly over the 32 mm wide band of adhesive. The IMMUNODYNE™ was then pressed against the film with a pressure of about 140 $g/cm^2$ (2 psi) to ensure uniform bonding to the film without damaging the pore structure of the IMMUNODYNE™.

The 20.3 cm (8 inch) length of the coated cellulose acetate with the IMMUNODYNE™ strip secured thereto was cut into strips 90 mm long and 8 mm wide with the IMMUNODYNE™ on one side of one end of the strip and having dimensions of 16 mm (length) by 8 mm (width).

The device of Example 1 was used to detect and quantify the presence of the antibody to the bovine hormone glucagon in fetal calf serum. The presence of an antibody to one of the animal's own secretions would be suggestive of an autoimmune disease. Typical examples of such diseases are rheumatoid arthritis, and lupus erythematosus. Detection and quantification of antibodies in fetal calf serum are illustrated in Examples 2-5.

**Example 2**

The end of one of the devices prepared as described in Example 1 bearing the membrane indicator medium or test field was immersed in a test tube containing 1.5 ml fetal calf serum for 90 minutes. Upon removal from the serum, the device was washed by agitating it (for 1 minute each time) in three successive 1.5 ml portions of PBS containing 0.1 percent Triton X-100, a non-ionic polyethoxylated nonyl phenol detergent available from the Rohm and Haas Co. It was then agitated (for 1 minute each time) in two successive 1.5 ml portions of PBS. The device was thereafter immersed for 90 minutes in 1.5 ml of PBS containing 190,000 counts per minute of $^{125}$I-labeled goat anti-rabbit IgG (product number NEX 155 of New England Nuclear). Upon removal from this solution, the device was washed by agitating it (for 1 minute each time) in three successive 1.5 ml portions of PBS containing 0.1 percent Triton X-100. The radioactivity remaining on the device was measured by standard procedures using a LKB/Wallace Mini Gamma gamma ray counter Model No. 1275. The device showed 2019 cpm (counts per minute) of residual gamma activity. These data are tabulated in Table I, as are the data from Examples 3-5 below.

**Example 3**

Another of the devices prepared as described in Example 1 was treated as described in Example 2, except that the fetal calf serum contained 0.004 microliters per milliliter of a commercial preparation of rabbit anti-(bovine glucagon) IgG (product number 969133 available from Calbiochem). When counted for residual radioactivity, the device showed 2302 cpm.

**Example 4**

A third of the devices prepared as described in Example 1 was treated as described in Example 2, except that the fetal calf serum contained 0.04 microliters of the rabbit anti-(bovine glucagon) IgG per milliliter of the fetal calf serum, i.e., the fetal calf serum had a concentration of the antibody ten times (10X) that of the fetal calf serum of Example 3. When counted for residual radioactivity, the device showed 2893 cpm.

**Example 5**

A fourth of the devices prepared as described in Example 1 was treated as described in Example 2, except that the fetal calf serum contained 0.4 microliters of the rabbit anti-(bovine glucagon) IgG per milliliter of the fetal calf serum, i.e., the fetal calf serum had a concentration 100 times (100X) that of the fetal calf serum of Example 3. When counted for residual radioactivity, the device showed 5747 cpm.

## TABLE I

| Device of Example | Antibody Concentration (µl/ml) | Counts/ Minute |
|---|---|---|
| 2 | 0 | 2019 |
| 3 | 0.004 | 2302 |
| 4 | 0.04 | 2893 |
| 5 | 0.4 | 5747 |

The data in Table I show that there is a unique correspondence between the radioactivity counted on the device and the amount of anti-glucagon antibody in the fetal calf serum. Such a one-to-one correspondence permits the radioactivity to be taken as an indication of the concentration of anti-glucagon antibody in the serum, demonstrating that the analytical procedure is quantitative.

**Example 6**

A device was fabricated as described in Example 1, except that the pore size of the IMMUNODYNE™ membrane indicator medium or test field used was 5.0 micrometers and the surface area was 3.24 m²/gram. This device was then treated as described in Example 2. When the device was counted for residual radioactivity, it showed 1698 cpm. This data is tabulated in Table II, as is the data from Examples 2 and 3 above and Example 7 below.

**Example 7**

A device was fabricated as described in Example 6. This device was then treated as described in Example 3. When the device was counted for residual radioactivity, it showed 1640 cpm.

## TABLE II

| Device Example | IMMUNODYNE™ Pore Size, micrometers | Antibody Concentra- tion (µl/ml) | Count/ Minute | Surface[1] Area (m²/g) |
|---|---|---|---|---|
| 2 | 0.2 | 0 | 2019 | 8.44 |
| 3 | 0.2 | 0.004 | 2302 | 8.44 |
| 6 | 5.0 | 0 | 1698 | 3.24 |
| 7 | 5.0 | 0.004 | 1640 | 3.24 |

[1]    Measured by a standard BET technique, as described in "Automated Instrument for Adsorption Desorption Isotherms" by J. E. Shields and S. Lowell, American Laboratory, November 1984, page 81, using an Autosorb-1 Model Sorptometer available from Quantachrome.

Table II illustrates the discernible difference in the radioactivity of the devices of Examples 2 and 3, indicating that when the diagnostic device was fabricated using a 0.2 micrometer IMMUNODYNE™ indicator medium or test field, the immunoassay was sensitive enough to detect the presence of antibody at a low concentration, i.e., 0.004 µl/ml. By contrast, when the device was fabricated using a 5.0 micrometer IMMUNODYNE™ indicator medium or test field (Examples 6 and 7), no significant difference was detected between the device exposed to an antibody concentration of zero and one exposed to a concentration of 0.004 µl/ml. Accordingly, when using a 5.0 micrometer IMMUNODYNE™ support, an antibody concentration as small as 0.004 µl/ml could not be detected. This demonstrates that for the glucagon antibody immunoassay a pore size of 0.2 micrometer in the membrane support is preferred over a pore size of 5.0 micrometer because of the increased sensitivity of the resultant assay. In this case, the largest reagent of the immunoassay system is of molecular dimensions and is expected to be able to thoroughly penetrate the pore structure of both the 0.2 micrometer and 5.0 micrometer membrane supports. It is believed that the increased sensitivity of the immunoassay performed with the 0.2 micrometer support is due to the greater surface area of that support compared with the surface area of the 5.0 micrometer support, i.e., 8.44 m²/gram versus 3.24 m²/gram.

The device in accordance with the present invention can also be used to detect antibodies to whole cells, as well as antibodies to biological molecules. In such cases, the pores of the membrane support in the device must be large enough to permit penetration of the cells (antigen) into the support. In this manner, maximum sensitivity to the antibody can be achieved. When the pore size is too small to permit penetration of the antigen, very small quantities of antibody cannot be detected.

**Claims**

1. A diagnostic device for testing a liquid to quantitatively detect the presence of biologically-active material in the liquid, said device comprising an elongated support member having secured to at least a portion thereof a liquophilic, microporous, synthetic, alcohol-insoluble, polyamide membrane or a liquophilic, microporous, synthetic, polyvinylidene difluoride membrane as an indicator medium or test field, the secured membrane having a surface area of from 2 to 12 $m^2$/gram, a pore rating of from 0.04 to 10 micrometers with a pore size distribution of no greater than ± 15% and the ability to immobilize a reagent thereon in a reproducible manner.

2. The diagnostic device of claim 1 wherein said membrane has immobilized thereon one or more reagents.

3. The diagnostic device of claim 1 wherein said membrane is nylon 66.

4. The diagnostic device of claim 1 wherein said membrane is a modified polyamide membrane with controlled surface properties.

5. The diagnostic device of claim 4 wherein said membrane is a nylon 66 membrane modified with a water-soluble polymer containing an abundance of surface carboxyl groups.

6. The diagnostic device of claim 5 wherein said modified membrane is treated with trichloro-s-triazine.

7. The diagnostic device of claim 6 wherein said medium has glucagon immobilized thereon.

8. The diagnostic device of claim 1 wherein said membrane has a pore rating of from 0.1 to 5 micrometers.

9. A diagnostic method for testing a liquid to quantitatively detect biologically-active material in the liquid comprising contacting the liquid containing the biologically-active material to be analyzed for with a liquophilic, microporous, synthetic membrane indicator medium or test field reagent immobilized thereon, said membrane comprised of an alcohol-insoluble polyamide or polyvinylidene difluoride and having a surface area of from 2 to 12 $m^2$/gram, a pore rating of from 0.04 to 10 micrometers with a pore size distribution no greater than ± 15% and the ability to immobilize the reagent thereon in a reproducible manner.

10. A method of preparing a diagnostic device for biologically-active materials comprising (1) treating an indicator medium or test field material to immobilize one or more test reagents thereon and (2) thereafter preparing a diagnostic device from a portion of the treated indicator medium or test field material.

**Patentansprüche**

1. Diagnostische Vorrichtung zum Testen einer Flüssigkeit für den quantitativen Nachweis der Anwesenheit eines biologisch aktiven Materials in der Flüssigkeit, wobei die Vorrichtung ein längliches Trägerelement aufweist, an dem zumindest in einem Teilbereich eine liquophile, mikroporöse, synthetische, in Alkohol unlösliche Polyamid-Membran oder eine liquophile, mikroporöse, synthetische Polyvinylidendifluorid-Membran als Indikatormedium oder Testfeld befestigt ist, wobei die befestigte Membran eine Oberfläche von 2 bis 12 $m^2$/g, eine Porengröße von 0,04 bis 10 $\mu$m mit einer Porengrößenverteilung von nicht mehr als ± 15 % aufweist und die Fähigkeit besitzt, daran ein Reagenz in reproduzierbarer Weise zu immobilisieren.

2. Diagnostische Vorrichtung nach Anspruch 1, wobei an der Membran eines oder mehrere Reagenzien immobilisiert worden sind.

3. Diagnostische Vorrichtung nach Anspruch 1, wobei es sich bei der Membran um Nylon 66 handelt.

**4.** Diagnostische Vorrichtung nach Anspruch 1, wobei es sich bei der Membran um eine modifizierte Polyamid-Membran mit kontrollierten Oberflächeneigenschaften handelt.

**5.** Diagnostische Vorrichtung nach Anspruch 4, wobei es sich bei der Membran um eine Nylon 66-Membran handelt, die mit einem wasserlöslichen Polymer mit einer reichlichen Menge an oberflächlichen Carboxylgruppen modifiziert ist.

**6.** Diagnostische Vorrichtung nach Anspruch 5, wobei die modifizierte Membran mit Trichlor-s-triazin behandelt ist.

**7.** Diagnostische Vorrichtung nach Anspruch 6, wobei an dem Medium Glucagon immobilisiert ist.

**8.** Diagnostische Vorrichtung nach Anspruch 1, wobei die Membran eine Porengröße von 0,1 bis 5 $\mu$m aufweist.

**9.** Diagnostisches Verfahren zum Testen einer Flüssigkeit zum quantitativen Nachweis eines biologisch aktiven Materials in der Flüssigkeit, umfassend das Kontaktieren der Flüssigkeit mit einem Gehalt an dem zu analysierenden biologisch aktiven Material mit einem liquophilen, mikroporösen, synthetischen Membran-Indikatormedium oder Testfeld mit daran immobilisiertem Reagenz, wobei die Membran ein in Alkohol unlösliches Polyamid oder Polyvinylidendifluorid umfaßt und eine Oberfläche von 2 bis 12 m$^2$/g, eine Porengröße von 0,04 bis 10 $\mu$m mit einer Porengrößenverteilung von nicht mehr als ± 15 % aufweist und die Fähigkeit besitzt, das Reagenz daran in reproduzierbarer Weise zu immobilisieren.

**10.** Verfahren zur Herstellung einer diagnostischen Vorrichtung für biologisch aktive Materialien, umfassend (1) die Behandlung eines Indikatormediums oder Testfeldmaterials, um daran eines oder mehrere Testreagenzien zu immobilisieren, und (2) die anschließende Herstellung einer diagnostischen Vorrichtung aus einem Teil des behandelten Indikatormediums oder Testfeldmaterials.

**Revendications**

**1.** Dispositif de diagnostic destiné au dosage d'un liquide pour détecter quantitativement la présence d'une substance biologiquement active dans le liquide, ledit dispositif comprenant un élément support allongé à au moins une partie duquel est fixée une membrane liquophile microporeuse synthétique en polyamide insoluble dans l'alcool ou une membrane liquophile microporeuse synthétique en difluorure de polyvinylidène comme milieu indicateur ou zone test, la membrane fixée ayant une aire de surface comprise entre 2 et 12 m$^2$/g, des pores de dimensions comprises entre 0,04 et 10 $\mu$m pour une distribution des dimensions des pores inférieure ou égale à ± 15 % et la capacité d'immobiliser un réactif de façon reproductible.

**2.** Dispositif de diagnostic selon la revendication 1, dans lequel ladite membrane porte un ou plusieurs réactifs immobilisés.

**3.** Dispositif de diagnostic selon la revendication 1, dans lequel ladite membrane est du nylon 66.

**4.** Dispositif de diagnostic selon la revendication 1, dans lequel ladite membrane est une membrane en polyamide modifiée ayant des propriétés de surface contrôlées.

**5.** Dispositif de diagnostic selon la revendication 4, dans lequel ladite membrane est une membrane en nylon 66 modifiée par un polymère hydrosoluble contenant une abondance de groupements carboxyle en surface.

**6.** Dispositif de diagnostic selon la revendication 5, dans lequel ladite membrane modifiée est traitée par la trichloro-s-triazine.

**7.** Dispositif de diagnostic selon la revendication 6, dans lequel ledit milieu porte du glucagon immobilisé.

**8.** Dispositif de diagnostic selon la revendication 1, dans lequel ladite membrane a des pores de dimensions comprises entre 0,1 et 5 $\mu$m.

EP 0 274 911 B1

**9.** Procédé de diagnostic destiné au dosage d'un liquide pour détecter quantitativement une substance biologiquement active dans le liquide, comprenant la mise en contact du liquide contenant la substance biologiquement active à analyser avec un milieu indicateur ou zone test constitué par une membrane synthétique liquophile microporeuse portant un réactif immobilisé, ladite membrane étant en un polyamide insoluble dans l'alcool ou en difluorure de polyvinylidène et ayant une aire de surface de 2 à 12 m$^2$/g, des pores de dimensions comprises entre 0,04 et 10 $\mu$m pour une distribution des dimensions des pores inférieure ou égale à ± 15 % et la capacité d'immobiliser le réactif de façon reproductible.

**10.** Procédé de préparation d'un dispositif de diagnostic pour substances biologiquement actives, comprenant (1) le traitement du matériau d'un milieu indicateur ou zone test pour y immobiliser un ou plusieurs réactifs de dosage et (2) la préparation subséquente d'un dispositif de diagnostic à partir d'une partie du matériau du milieu indicateur ou zone test traité.